(19) Europäisches Patentamt
European Patent Office
Office européen des brevets

(11) **EP 2 123 770 A1**

(12) **EUROPEAN PATENT APPLICATION**

(43) Date of publication:
**25.11.2009 Bulletin 2009/48**

(51) Int Cl.:
*C12Q 1/02* (2006.01)     *G01N 33/50* (2006.01)

(21) Application number: **08380156.3**

(22) Date of filing: **19.05.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS** | (72) Inventors:<br>• **Tarón Roca, Miguel**<br>  **08028 Barcelona (ES)**<br>• **Rosell Costa, Rafael**<br>  **08028 Barcelona (ES)** |
| (71) Applicant: **Pangaea Biotech, S.A.**<br>**08028 Barcelona (ES)** | (74) Representative: **ABG Patentes, S.L.**<br>**Avenida de Burgos 16D**<br>**Edificio Euromor**<br>**28036 Madrid (ES)** |

(54) **Method for determining the DNA repair capacity of a cell**

(57)    The invention relates to methods for determining the inherited or acquired DNA repair capacity of a cell or cell population, and to the use of said method for determining the genetic susceptibility to cancer of a subject or for predicting the response of a subject suffering from cancer to a chemotherapy treatment directed to damaging the DNA.

These methods involve the use of a polynucleotide encoding in its natural form a fluorescent protein (e.g. EGFP). Said polynucleotide has been previously contacted with a genotoxic agent (e.g. UV), rendering said polynucleotide unable to express a functional fluorescent protein. Host repair system capacity will be assessed by fluorescence.

EP 2 123 770 A1

**Description**

**FIELD OF THE INVENTION**

The present invention relates to the field of diagnostics and, in particular, to a method for determining the inherited or acquired DNA repair capacity of a cell or cell population, e.g. peripheral blood leukocytes cells (PBLs), and to the use of said method for determining the genetic susceptibility to cancer of a subject or for predicting the response of a subject suffering from cancer to a chemotherapy treatment directed to damaging the DNA.

**BACKGROUND OF THE INVENTION**

There is a growing awareness that many human cancers result from the combined effects of environmental exposure and inherent susceptibility states. Humans display a considerable range of clinical sensitivities to environmental agents such as radiation and chemical carcinogens. This variability in host response is believed to be due, at least in part, to inherent differences between individuals to repair damaged sites induced in their genetic material by many of these environmental agents.

A lowered DNA repair capability may promote the malignant transformation of target cells within the exposed individual and ultimately increase the likelihood that neoplasia occurs.

Excision appears to be the most prevalent form of DNA repair in human cells. This comprises a series of coordinated enzymatic steps through which damaged sites are excised from DNA along with neighbouring single strand regions, and the resulting gap is re-synthesized using the intact opposite strand as a template. Thus, through the concerted action of various cellular repair enzymes, potentially oncogenic/cytotoxic lesions are removed from DNA and native biologic structure and function subsequently restored.

Since the initial incision of DNA near the site of damage is considered a crucial, rate-limiting step in the overall repair process, measuring the rate or extent to which a given cell population is able to incise damaged DNA, or perform any subsequent step, is generally considered indicative of repair proficiency. Although many procedures are capable of providing such measures, most require technical capabilities which preclude their use on a routine large-scale basis. Thus, existing methodology for population-based assessment of cellular repair essentially is limited to examining the extent to which UV-exposed lymphocytes incorporate the radiolabeled nucleoside, [$^3$H]-thymidine, into UV-damaged cellular DNA during the re-synthesis step. Commonly referred to as unscheduled DNA synthesis (UDS), the assay achieves a quantitative measure through scintillation counting of intact cells or cell extracts following a post-irradiation repair incubation period. Although capable of detecting large differences, a number of confounding biochemical artefacts exist in UDS which significantly reduce the specificity with which relatively smaller differences in incorporated radioactivity (as would be expected in the general population) may reflect true differences in repair proficiency. Furthermore, since entire cells are being irradiated, the introduction of uniform amounts of cellular DNA damage between large numbers of replicate and independent cell cultures cannot be easily controlled, thus effectively lowering both assay precision and accuracy.

For purposes of analytical epidemiologic research, a laboratory procedure for screening cellular repair proficiency optimally should be highly sensitive and specific for DNA repair; quantitative; precise; routine; and relatively inexpensive. Since UDS is deficient in several of these characteristics, with fundamental components of the assay being involved, modification of this procedure to render it appropriate for epidemiologic study is not considered feasible.

Recent advances in recombinant technology allowing for large scale molecular cloning, transfer and expression of biologically active DNA in cultured human cells have opened up the possibility of designing procedures for measuring cell repair potential. It has also been noted that exogenously damaged DNA, when introduced into cultured human cells, is acted upon by cellular repair enzymes. This phenomena, termed host cell reactivation (HCR), is based on findings that human "host" cells, after infection with radiation or chemically damaged (i.e., inactivated) viral particles, were capable of reactivating the inactivated virus to a biologically competent state as measured by plaque formation, viral antigen formation, cell transformation, etc. The reactivation of biologic competence is taken as an indirect measure of cell ability to restore functionality to damaged regions of the viral genome, a direct consequence of DNA repair.

US patent US4975365 discloses a quantitative host cell reactivation assay method comprising (i) providing a recombinant DNA plasmid containing a bacterial gene which is expressed in cells to be tested for cell repair proficiency and whose respective product is amenable to screening; (ii) inactivating the gene by a genotoxic agent; (iii) transfecting the cells to be tested with the thus inactivated gene; and (iv) determining the repair efficiency of said cells by comparing the percent gene expression with the percent gene expression obtained when the same DNA plasmid without inactivation is transfected into the cells. The method may be used to test repair potential in fresh human lymphocytes or in amniotic cells.

Qiao, Y. et al., (Mutation Research, 2002, 509:165-175) disclose a modified host-cell reactivation assay using luciferase reporter gene in order to establish a correlation between DNA repairing capacity of a cell and the presence of polymorphisms in DNA repair genes. However, this method is unspecific since it does not allow to distinguish between dead and alive cells.

Laget, M et al., (Path Biol., 1995, 43:596-600) disclose an assay for determining the DNA repair capacity based on flow cytometry, wherein the cells treated with genotoxic agents (mitomycin C and adriblastine) are fractionated by means of flow cytometry depending on DNA synthesis. The DNA repair capacity is indirectly determined based on the assumption that the cells with damaged DNA and without capacity for repairing it are incapable of going ahead with the cellular cycle which results in a different amount of cellular DNA.

Therefore, there is a need in the art for methods suitable for the determination of the inherited or acquired DNA repair capacity of a cell or cell population in a more specific and stable manner than the conventional methods.

## SUMMARY OF THE INVENTION

In a first aspect, the invention relates to an *in vitro* method for determining the DNA repair capacity of a cell comprising:

(i) introducing into said cell a polynucleotide encoding in its natural state a fluorescent protein, wherein said polynucleotide has been previously contacted with a genotoxic agent so that the polynucleotide has been rendered unable of expressing a functional fluorescent protein;
(ii) maintaining the cells under conditions adequate for expression of said polynucleotide and
(iii) determining the fluorescent emission of the cell due to the fluorescent polypeptide

wherein the intensity of the fluorescence emission is indicative of the DNA repair capacity of the cell.

In a second aspect, the invention relates to an *in vitro* method for determining the DNA repair capacity of a cell population comprising:

(i) introducing into said cell population a polynucleotide encoding in its natural state a fluorescent protein, wherein said polynucleotide has been previously contacted with a genotoxic agent so that the polynucleotide has been rendered unable of expressing a functional fluorescent protein;
(ii) maintaining the cells under adequate conditions for expression of said polynucleotide and
(iii) determining the percentage of cells emitting fluorescence due to the fluorescent polypeptide above a threshold fluorescence value,

wherein the threshold fluorescence value is the value of a control cell population which does not contain a polynucleotide coding for a fluorescent protein and wherein the percentage of cells emitting fluorescence over said threshold level is indicative of the DNA repair capacity of the cell population.

In a third aspect, the invention refers to an isolated polynucleotide encoding in its natural state a fluorescent protein, wherein said polynucleotide has been contacted with a genotoxic agent so that (i) the polynucleotide has been rendered unable to express a fluorescent protein and (ii) the polynucleotide comprises, at least, one thymidine dimer.

In a fourth aspect, the invention relates to an *in vitro* method for determining the genetic susceptibility to cancer of a subject comprising

(i) determining the DNA repair capacity of a cell from said subject or of a cell population from said subject using the methods of the invention and
(ii) comparing the DNA repair capacity values obtained in step (i) with standard reference values

wherein a DNA repair capacity lower than the DNA repair capacity of the reference values is indicative of high susceptibility to cancer.

Finally, in a fifth aspect, the invention relates to an *in vitro* method for predicting the response of a subject suffering from cancer to a chemotherapy treatment directed to damaging DNA comprising

(i) determining the DNA repair capacity of a cell or of a cell population from said subject by a method of the invention, and
(ii) comparing the DNA repair capacity obtained in step (i) with standard reference values

wherein a DNA repair capacity values lower than the DNA repair capacity of the reference values is indicative of a better response of the subject to the chemotherapy directed to damaging DNA.

**BRIEF DESCRIPTION OF THE FIGURES**

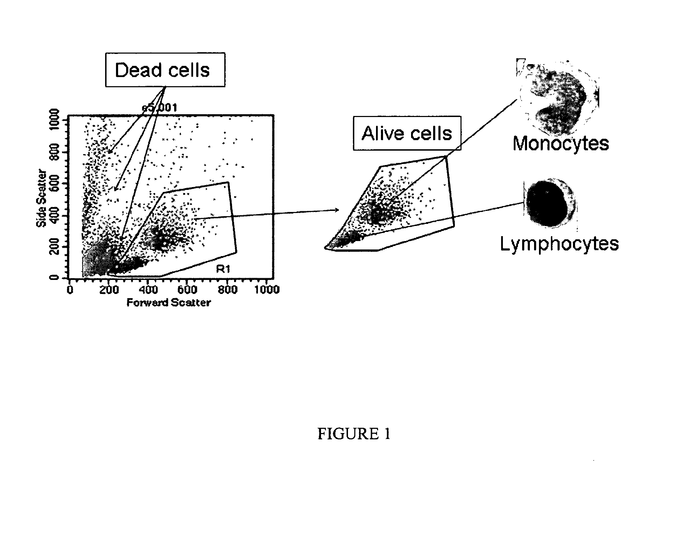

Figure 1 is a FACS scan of an isolated PBL population detected using side scatter (ordinate) and forward scatter (abscissa). The scan allows the identification of three different cell populations. Dead cells showing high side scatter values, lymphocytes showing low side scatter and low forward scatter values and monocytes showing higher side scatter and forward scatter values than the lymphocytes. The region within the plot comprises the alive cells of the population (lymphocytes and monocytes).

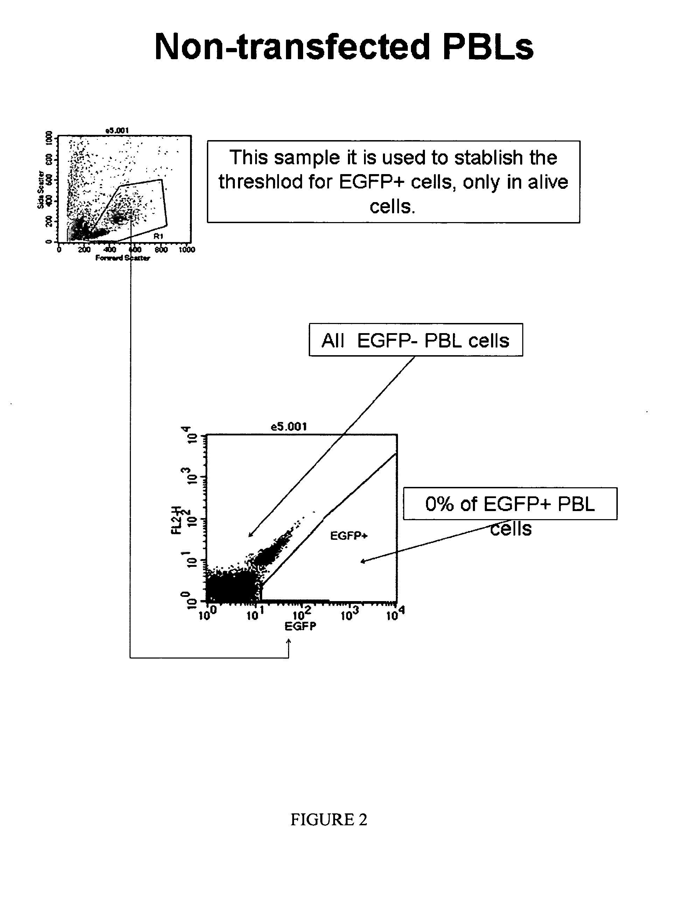

Figure 2 is a dual-excitation FACS scan using FL2H to detect autofluorescence (PE filter with a maximum emission of λ=578 nm in the ordinate) and EGFP filters (abscissa) to detect EGFP emission obtained using the viable cell population obtained in figure 1. The line in the FACS scan represents the threshold established by using non-transfected cells from the same patient in order to distinguish cells positive for EGFP from cells negative for EGFP.

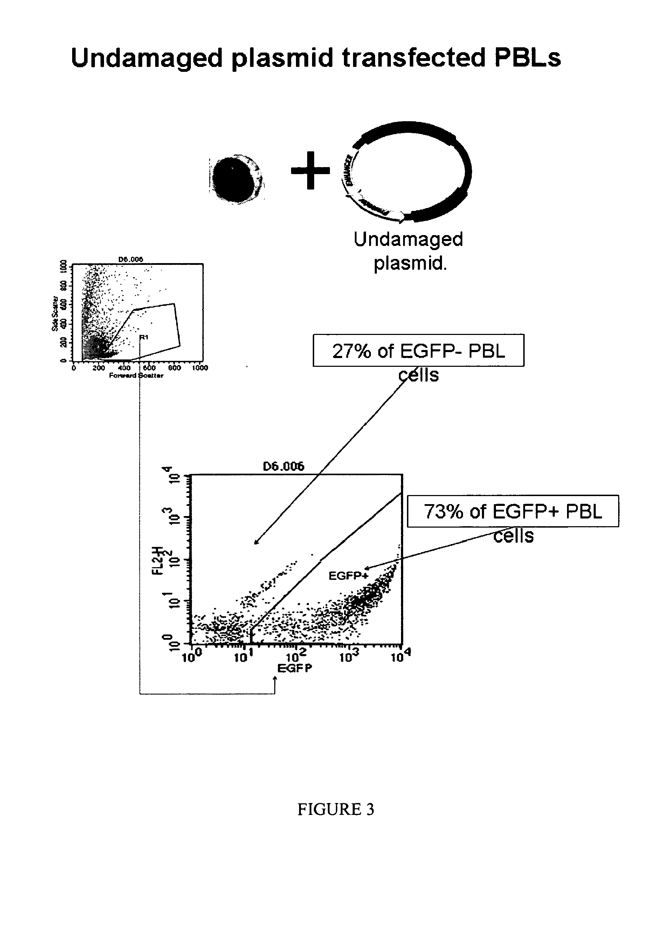

Figure 3 is a dual-excitation FACS scan using FL2H (ordinate) and EGFP (abscissa) filters from a cell viable population derived from PBL selected as described in figure 1 and which has been transfected with an undamaged plasmid encoding EGFP. The FACS scan shows that that 27% of the PBLs population was negative to EGFP (i.e. it does not display fluorescence above the threshold value as determined in figure 2) whereas the 73% of the population was positive to EGFP (i.e. it displays fluorescence above a threshold value selected as in figure 2). The percentage of EGFP+ cells from this transfected with undamaged-DNA plasmid sample (73%) will be used as reference of maximum expression of EGFP gene in this sample/patient.

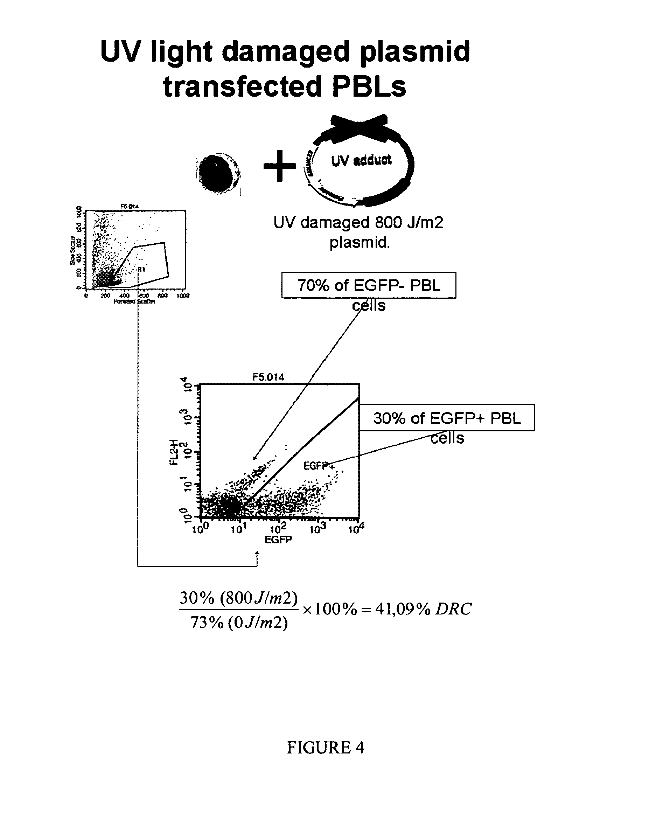

Figure 4 is a dual-excitation FACS scan using FL2H (ordinate) and EGFP (abscissa) filters from a cell viable population derived from PBL selected as described in figure 1 and which has been transfected with an plasmid encoding EGFP previously damaged using UV-light at 800 J/m$^2$. The FACS scan shows that 70% of the PBLs population was negative to EGFP (i.e. it does not display fluorescence above the threshold value as determined in figure 2) whereas 30% of the population was positive to EGFP (i.e. it displays fluorescence above a threshold value selected as in figure 2). The percentage of EGFP+ cells in the DNA-damaged plasmid transfected cells will be used as the percentage of cells that were able to reactivate (Repair) the expression of the fluorescent protein coding polynucleotide. The formula indicates that the DNA repair capacity was determined by dividing the percentage of cells emitting fluorescence above the threshold value (30%) in damaged-DNA transfected sample by the percentage of cells emitting fluorescence above the threshold level (73%) when transfected with an undamaged EGFP-encoding plasmid.

**DETAILED DESCRIPTION OF THE INVENTION**

The authors of the present invention have developed a new method for determining the DNA repair capacity (DRC) of a cell based on the conventional "host cell reactivation assay" (HCRA). Particularly, the inventors have found that using polynucleotide encoding a fluorescent protein as reporter gene and detecting its activity by means of flow cytometry allows, surprisingly, the determination of the DNA repair capacity of a cell in a more specific and stable manner than the conventional HCRA.

Thus, in one aspect, the present invention refers to an *in vitro* method (hereinafter the first method of the invention) for determining the DNA repair capacity of a cell comprising:

(i) introducing into said cell a polynucleotide encoding in its natural state a fluorescent protein, wherein said polynucleotide has been previously contacted with a genotoxic agent so that the polynucleotide has been rendered unable of expressing a functional fluorescent protein;
(ii) maintaining the cells under conditions adequate for expression of said polynucleotide and
(iii) determining the fluorescent emission of the cell due to the fluorescent polypeptide

wherein the intensity of the fluorescence emission is indicative of the DNA repair capacity of the cell.

The term "DNA repair capacity" or "DRC", as used herein, relates to the capacity of a cell to repair damaged sites in the DNA.

The DNA repair capacity of a cell can be "inherited" or "acquired". Nevertheless, the first method of the invention can be used to determine both the "inherited" DNA repair capacity and the "acquired" DNA repair capacity of a cell.

The term "inherited DNA repair capacity", as used herein, relates to the DNA repair capacity genetically inherit by the host from their parents.

The term "acquired DNA repair capacity", as used herein, relates to the inherited DNA repair capacity somatically modified during the lifespan, due to smoking habit, somatic negative/positive mutations, etc.

The method of the invention requires introducing into a cell a polynucleotide encoding in its natural state a fluorescent protein. A polynucleotide is an organic polymer molecule composed of nucleotide monomers covalently bonded in a chain. In the context of the present invention, the polynucleotide is DNA (deoxyribonucleic acid).

Standard procedures may be used for delivering DNA to the interior of the cells (see sections 9.1 to 9.5 in Ausubel, F.M. et al., Current Protocols in Molecular Biology, John Wiley & Sons Inc; ringbou edition, 2003). Suitable methods for introducing DNA into a cell, for example, include precipitating DNA with calcium phosphate, DEAE-dextran, polybrene, electroporation, microinjection, liposome-mediated fusion, lipofection, biolistic transfection, infection by viral vectors, etc.

In a particular embodiment, the DNA delivering to the interior of the cells is carried out by electroporation, for example, as described in M.L. Roach and J.D. McNeish (2002) (Methods in Mol. Biol., 2002, 185:1). For this purpose, cells are placed in suspension in an appropriate electroporation buffer and placed into an electroporation cuvette. DNA is added, the cuvette is connected to a power supply, and the cells are subjected to a high-voltage electrical pulse of defined magnitude and length. The cells are then allowed to recover briefly before they are placed in normal growth medium.

As the skilled person understands, the overall efficiency of the electroporation procedure depends collectively on numerous key parameters, including DNA concentration, purity and topology; carrier DNA; and electrical conditions such as ionic strength of the electroporation buffer, electric field strength, pulse duration and capacitance. Individual methodologies that address each one of these parameters are known in sufficient detail in the art to enable successful reproduction of this method.

In another particular embodiment, the electroporation is carried out by nucleofection. Nucleofection is an electroporation-based transfection method that enables the DNA to enter directly the nucleus, given rise to a fast, easy and highly effective alternative method for the conventional electroporation.

As the skilled person understands, the polynucleotide or DNA to be introduced into the cell may be comprised into a vector, being said vector a viral vector or a non-viral vector, such as a plasmid. A vector is any vehicle used to transfer foreign genetic material into another cell.

By way of a non-limiting illustration, said vectors can be viral vectors based on retroviruses, adenoviruses, alphaviruses, etc., or in case of non-viral vectors, the vectors can be DNA-liposome, DNA-polymer, DNA-polymer-liposome complexes, pSilencer 4.1-CMV (Ambion), pcDNA3, pcDNA3.1/hyg pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d, pTDT1, pmaxFP-G-C, pmaxFP, etc. Said viral and non-viral vectors comprising the polynucleotide encoding in its natural state a fluorescent protein can be administered to the cell by the conventional methods previously cited.

The vector can contain, among others, multiple cloning sites, expression regulating sequences, suitable replication origins for the host cell in which the vector is introduced, selection markers, etc. In the context of the present invention, the vector is an expression vector which allows the expression of the polynucleotide in the target cell and generally has a promoter sequence (or expression regulating sequences) that drives expression thereof. The promoter sequence preceding the polynucleotide is operatively bound to the said polynucleotide. As used in this description, the expression "operatively bound" means that the polynucleotide is within the correct reading frame for their expression under the control of said regulating sequences.

The regulating sequences useful for the present invention can be nuclear promoting sequences or, alternatively, enhancer sequences and/or other regulating sequences increasing the expression of the polynucleotide. The promoter can be constitutive or inducible. If the constant expression of the polynucleotide is desired, then a constitutive promoter is used. Examples of well known constitutive promoters include the cytomegalovirus (CMV) immediate-early promoter, the Rous sarcoma virus promoter, and the like. A number of other examples of constitutive promoters are well known in the art and can be used in implementing the invention. If the controlled expression of the polynucleotide is desired, then an inducible promoter must be used. In a non-induced state, the inducible promoter must be "silent". "Silent" is understood to mean that in the absence of an inducer, little or no expression of the polynucleotide is detected; in the presence of an inducer, however, the expression of the polynucletide occurs. The expression level can frequently be controlled by varying the concentration of the inducer so that the inducible promoter is stimulated more strongly or weakly and consequently, the concentration of the polynucletide transcribed product is affected. Examples of well-known inducible promoters are: an androgen or estrogen-responsive promoter, a doxycicline-responsive promoter, a metallothionein promoter, or a promoter responding to ecdysone. Other various examples are well known in the art and can be used for implementing the invention.

In the context of the present invention, the polynucleotide to be used encodes in its natural state a fluorescent protein and can comprise, or not, the promoter or the regulating sequences. The expression "in its natural state" means that the polynucleotide occurs in nature, i.e. naturally occurring polynucleotide which has not been altered by the hand of the man.

Any member of the fluorescent protein family can be used, including, non limiting to, green fluorescent protein (GFP), cyan fluorescent protein (CFP), red fluorescent protein (RFP), yellow fluorescent protein (YFP), blue fluorescent protein (BFP), orange fluorescent protein (OFP), etc. In a particular embodiment, the fluorescent protein is enhanced green fluorescent protein (EGFP).

According to step (i) of the first method of the invention, the polynucleotide encoding in its natural state a fluorescent protein and which can be comprised, or not, into a vector, has to be contacted with a genotoxic agent. The term "genotoxic agent" relates to a physical, biological or chemical agent capable of inducing a lesion in the DNA. As the skilled person understands, typical DNA lesions include:

- Lesions of purine or pyrimidine bases: oxidative lesions induced by the cellular metabolism and by photosensitization; lesions through the formation of chemical adducts, which result from the harmful action of many genotoxic agents, such as polycyclic hydrocarbons, contained in combustion products; lesions through the formation of metheno-bases or of etheno-bases;
- Lesions of the 3D-structure of the DNA double helix: formation of intrastrand (between two adjacent bases of the same strand) or interstrand (between two bases located on the homologous strands) bridges, generally caused by ultraviolet radiation (formation of bridges between pyrimidines, which become dimeric) or bifunctional antitumor agents, such as cisplatin and intercalating agents, which form stable covalent bonds between the bases carried by opposite strands;
- Lesions through single-stranded or double-stranded breakage: produced by agents such as ionizing radiation and through the action of free radicals; and
- Sugar lesions: the destruction of a deoxyribose results in breakage of phosphodiester bonds in the damaged site, followed by strand breakage.

Agents capable of inducing a lesion in the DNA include physical, biological and chemical agents. Physical agents capable of inducing a lesion in the DNA are, for example, ultraviolet (UV) or ionizing radiation. Biological agents capable of inducing a lesion in the DNA are, for example, fungal and bacterial toxins. Chemical agents capable of inducing a lesion in the DNA are, for example, base analogs, such as 5-bromuracil, 2-aminopurine, etc; acridines, such as 2,8-diamino acridine (proflavin), etc.; alkylating agents, such us, ethylmethane sulfonate (EMS); deaminating agents such us nitrous acid; hydroxylamine and free radicals, etc. as well as those belonging to the carcinogen family, for example, acetylaminofluorene (Hess et al., 1996, Nucleic Acid Res. 24, 824-828), cisplatin (Pasheva et al., 2002, Int. J. Biochem. Cell Biol., 34, 87-92), benzopyrene (Laws et al., 2001, Mut. Res.; 484, 3-18), psoralen (Zhang et al., Mol. Cell. Biol., 2002, 22, 2388-2397), chloroacetaldehyde (CAA) (Wang et al., 2002, 13, 1149-1157), and tamoxifen (Dasaradhi et al., 1997, Chem. Res. Tox., 10, 189-196)

If desired, the polynucleotide or DNA encoding a fluorescent protein may be isolated from the vector, damaged and then reinserted into the vector. In the context of the present invention, when a genotoxic agent induces a lesion in the polynucleotide or DNA encoding a fluorescent protein, said lesion can have two important consequences: (1) the polynucleotide or DNA is not expressed if the lesion affects the promoter or regulating sequences or the damaged DNA does not serve as template for the RNA polymerase and (2) the fluorescent protein resulting from the expression of said polynucleotide or DNA is inactive. In both cases, the fluorescent protein can not perform its natural function, i.e., the protein can not emit light when is excited.

Step (ii) of the first method of the invention requires maintaining the cells under adequate conditions so that the polynucleotide introduced in step (i) can be expressed. As the skilled person will understand, "adequate conditions" include suitable conditions regarding temperature, pH, nutrients, and the like so that the polynucleotide can be expressed. Step (iii) of the first method of the invention comprises a step of determining the fluorescence of the cell due to the fluorescent marker. The conditions adequate for detecting the fluorescence of the fluorescent protein encoded by the damaged polynucleotide will obviously depend on the particular type of fluorescent protein used (see Table 1).

Table 1

| Protein (Acronym) | Excitation Maximum (nm) | Emission Maximum (nm) | Molar Extinction Coefficient | Quantum Yield | *in vivo* Structure | Relative Brightness (% of EGFP) |
|---|---|---|---|---|---|---|
| GFP (wt) | 395/475 | 509 | 21,000 | 0.77 | Monomer* | 48 |
| Green Fluorescent Proteins | | | | | | |
| EGFP | 484 | 507 | 56,000 | 0.60 | Monomer* | 100 |
| AcGFP | 480 | 505 | 50,000 | 0.55 | Monomer* | 82 |
| TurboGFP | 482 | 502 | 70,000 | 0.53 | Monomer* | 110 |
| Emerald | 487 | 509 | 57,500 | 0.68 | Monomer* | 116 |

(continued)

| Protein (Acronym) | Excitation Maximum (nm) | Emission Maximum (nm) | Molar Extinction Coefficient | Quantum Yield | *in vivo* Structure | Relative Brightness (% of EGFP) |
|---|---|---|---|---|---|---|
| Azami Green | 492 | 505 | 55,000 | 0.74 | Monomer | 121 |
| ZsGreen | 493 | 505 | 43,000 | 0.91 | Tetramer | 117 |
| **Blue Fluorescent Proteins** | | | | | | |
| EBFP | 383 | 445 | 29,000 | 0.31 | Monomer* | 27 |
| Sapphire | 399 | 511 | 29,000 | 0.64 | Monomer* | 55 |
| T-Sapphire | 399 | 511 | 44,000 | 0.60 | Monomer* | 79 |
| **Cyan Fluorescent Proteins** | | | | | | |
| ECFP | 439 | 476 | 32,500 | 0.40 | Monomer* | 39 |
| mCFP | 433 | 475 | 32,500 | 0.40 | Monomer | 39 |
| Cerulean | 433 | 475 | 43,000 | 0.62 | Monomer* | 79 |
| CyPet | 435 | 477 | 35,000 | 0.51 | Monomer* | 53 |
| AmCyan1 | 458 | 489 | 44,000 | 0.24 | Tetramer | 31 |
| Midori-Ishi Cyan | 472 | 495 | 27,300 | 0.90 | Dimer | 73 |
| mTFP1 (Teal) | 462 | 492 | 64,000 | 0.85 | Monomer | 162 |
| **Yellow Fluorescent Proteins** | | | | | | |
| EYFP | 514 | 527 | 83,400 | 0.61 | Monomer* | 151 |
| Topaz | 514 | 527 | 94,500 | 0.60 | Monomer* | 169 |
| Venus | 515 | 528 | 92,200 | 0.57 | Monomer* | 156 |
| mCitrine | 516 | 529 | 77,000 | 0.76 | Monomer | 174 |
| YPet | 517 | 530 | 104,000 | 0.77 | Monomer* | 238 |
| PhiYFP | 525 | 537 | 124,000 | 0.39 | Monomer* | 144 |
| ZsYellow1 | 529 | 539 | 20,200 | 0.42 | Tetramer | 25 |
| mBanana | 540 | 553 | 6,000 | 0.7 | Monomer | 13 |
| **Orange and Red Fluorescent Proteins** | | | | | | |
| Kusabira Orange | 548 | 559 | 51,600 | 0.60 | Monomer | 92 |
| mOrange | 548 | 562 | 71,000 | 0.69 | Monomer | 146 |
| dTomato | 554 | 581 | 69,000 | 0.69 | Dimer | 142 |
| dTomato-Tandem | 554 | 581 | 138,000 | 0.69 | Monomer | 283 |
| DsRed | 558 | 583 | 75,000 | 0.79 | Tetramer | 176 |
| DsRed2 | 563 | 582 | 43,800 | 0.55 | Tetramer | 72 |
| DsRed-Express (T1) | 555 | 584 | 38,000 | 0.51 | Tetramer | 58 |
| DsRed-Monomer | 556 | 586 | 35,000 | 0.10 | Monomer | 10 |
| mTangerine | 568 | 585 | 38,000 | 0.30 | Monomer | 34 |
| mStrawberry | 574 | 596 | 90,000 | 0.29 | Monomer | 78 |
| AsRed2 | 576 | 592 | 56,200 | 0.05 | Tetramer | 8 |
| mRFP1 | 584 | 607 | 50,000 | 0.25 | Monomer | 37 |
| JRed | 584 | 610 | 44,000 | 0.20 | Dimer | 26 |
| mCherry | 587 | 610 | 72,000 | 0.22 | Monomer | 47 |
| HcRed1 | 588 | 618 | 20,000 | 0.015 | Dimer | 1 |
| mRaspberry | 598 | 625 | 86,000 | 0.15 | Monomer | 38 |
| HcRed-Tandem | 590 | 637 | 160,000 | 0.04 | Monomer | 19 |
| mPlum | 590 | 649 | 41,000 | 0.10 | Monomer | 12 |
| AQ143 | 595 | 655 | 90,000 | 0.04 | Tetramer | 11 |

(continued)

| Protein (Acronym) | Excitation Maximum (nm) | Emission Maximum (nm) | Molar Extinction Coefficient | Quantum Yield | *in vivo* Structure | Relative Brightness (% of EGFP) *Weak Dimer |
|---|---|---|---|---|---|---|

Any suitable method for measuring the fluorescence of a substance can be used in the present invention. The fluorescence can be monitored, for example, by fluorescence microscopy and flow cytometry. If there is no need to physically sort the cells or to monitor intracellular migration, microplate fluorometry offers a preferable, more convenient and higher-throughput detection system. Also, the Analyst GT multimode reader from Molecular Devices can easily and non-invasively measure fluorescent proteins in viable cells. In a particular embodiment, the fluorescence detection is carried out by flow cytometry.

The fluorescence value of the cell population is then taken as a measure of the DRC of the cell. As it will be understood by a person skilled in the art, the fluorescence value can be compared to a reference value in order to determine the extent of the DRC of the cell. Preferably, the reference value is the fluorescence intensity of a control cell which has not been transformed with a plasmid encoding a fluorescent protein. Thus, a cell is considered to be able to repair the DNA whenever its fluorescence intensity is at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value obtained using a cell which has not been transformed.

As the skilled person understands, the method of the invention may comprise additional steps in order to optimize the results. Thus, if desired, the method comprises an additional step previous to the introduction into the cell of a polynucleotide, said step comprising culturing the cells in complete DMEM plus glucose culture media in order that the number of cells grows, preferably, 4,5 g/L glucose culture media.

The method developed in the present invention can be use for determining the DNA repair capacity (DRC) of a cell or a cell population.

Thus, in another aspect, the invention relates to an *in vitro* method (hereinafter the second method of the invention) for determining the DNA repair capacity of a cell population comprising:

(i) introducing into said cell population a polynucleotide encoding in its natural state a fluorescent protein, wherein said polynucleotide has been previously contacted with a genotoxic agent so that the polynucleotide is no longer able to express a functional fluorescent protein;
(ii) maintaining the cells under conditions adequate for expression of the polynucleotide and
(iii) determining the percentage of cells emitting fluorescence due to said fluorescent polypeptide above a threshold fluorescence value,

wherein said threshold fluorescence value is the fluorescence value of a cell population not containing the polynucleotide encoding a fluorescence protein and wherein the percentage of cells emitting above a certain threshold is indicative of the DNA repair capacity of the cell.

The DNA repair capacity of a cell population can be "inherited" or "acquired". Nevertheless, the second method of the invention can be used to determine both the "inherited" DNA repair capacity and the "acquired" DNA repair capacity of a cell population.

In the context of the present invention, the meaning of "DNA repair capacity" or "DRC", "inherited DNA repair capacity", "acquired DNA repair capacity", "natural state" and "natural function" has been previously explained.

As used herein, the term "threshold fluorescence value" is understood as the fluorescence value of a control cell population processed under identical conditions but which do not contain a polynucleotide encoding in its natural state a fluorescent protein. A way of determining a "threshold fluorescence value" is shown in the results of the Example following the detailed description of the invention and in Figure 2.

Cells populations adequate for performing the second method of the invention include any cell population which can be isolated from the subject. Preferably, cell populations adequate for detecting the DRC are those populations meeting at least one of the requirements (i) cell populations which can be obtained from the individual under minimally invasive conditions, (ii) cell populations which can be easily disaggregated so that the samples can be easily analysed by FACS and (iii) cell populations that are amenable of incorporating an heterologous DNA when placed in the presence of said DNA under adequate conditions. Preferably, the cell population is a blood sample and, more preferably, a sample of peripheral blood cells.

As the skilled person understands, all the methods cited above suitable for introducing a polynucleotide into a cell are applicable to the present method. Thus, in a particular embodiment, the delivering of a polynucleotide into a cell is carried out by transfection, preferably by means of electroporation. In another particular embodiment, the electroporation is carried out by nucleofection.

In a preferred embodiment, the second method of the invention includes in step (iii) the determination of the DNA repair capacity by dividing the percentage of cells emitting fluorescence above the threshold value in damaged-DNA transfected sample by the percentage of cells emitting fluorescence above the threshold level when transfected with an undamaged EGFP-encoding plasmid.

If desired, an additional step previous to step (i) of the second method of the invention can be added to the present method. The additional step comprises selecting the viable cells from the cell population and using the said cells in step (i) of the method. Suitable methods for selecting the viable from the dead cells are widely known in the art such as, for example, performing a sorting of viable cells based on forward and side scatter or using propidium iodide as dye-exclusion viability probe, which stains only necrotic cells and sorting them by fluorescence-activated cell sorting (FACS) [Steinkamp J., et al. (1999) Journal of Immunological Methods, 226(1-2): 59-70]; using coating matrix kit or collagen to get up viable cells adhered while the dead cells could not attach on it; Annexin V protein membrane immune-fluorescent staining for detecting apoptotic cells [Derby E, (2001) et al Immunol Lett. Aug 1;78(1):35-9]; etc.

All the different types of fluorescent proteins that can be used in the second method of the invention as well as the methods for detecting fluorescence are those previously disclosed for the first method of the invention. However, in a particular embodiment, the fluorescent protein is EGFP and, in another particular embodiment, the fluorescence detection is carried out by flow cytometry.

As previously explained, the polynucleotide or DNA to be introduced into the cell or cell population may be comprised into a vector, being said vector a viral vector or a non-viral vector, such as a plasmid.

Thus, in another aspect, the invention relates to a vector comprising a polynucleotide encoding in its natural state a fluorescent protein, wherein said polynucleotide has been contacted with a genotoxic agent so that (i) the protein can not perform its natural function and (ii) the polynucleotide comprises, at least, one thymidine dimmer.

The meaning of the term "natural function" and "natural state" in the context of the present invention has been already defined.

The term "thymidine dimer", as used herein, is understood as is the covalent bonding of two adjacent thymine residues within a DNA molecule

The presence of thymidine dimers in a polynucleotide can be determined using standard methodology such as:

- treatment with T4 endonuclease V which exhibits a N-glycosylase activity at the 5'-extremity of the lesion, leading to the formation of strand breaks which can be quantified using electrophoretic techniques;
- separating the DNA molecules using a combination of HPLC (High Perfomance Liquid Chromatography) and electro spray ionization tandem mass spectrometry (HPLC-MS/MS) [Douki, T. et al., 2000 J. Biol. Chem., 275:1167-11685];
- reacting the nucleic acid with KMn04 [Ramaiah, D. et al. 1998, Nucleic Acids Research, 26:3940-3943] and
- detection using thymidine dimmer-specific monoclonal antibodies [Komatsu, Y., et al. 1997, Nucleic Acids Research, 25:3889-3894; Lund, V. et al., 1989, Nucleic Acids Research, 17:539-551].

The polynucleotides of the invention can be used in isolated manner or forming part of a vector. Examples of viral vectors and non-viral vectors suitable for this invention have been disclosed in previous paragraphs. The vector can contain, for example, multiple cloning sites, expression regulating sequences, suitable replication origins for the host cell in which the vector is introduced, selection markers, etc. In the context of the present invention, the vector is an expression vector which allows the expression of the polynucleotide in the target cell. Suitable expression regulating sequences have been also disclosed previously in the present invention.

In a particular embodiment of the vector, the fluorescent protein is EGFP and, in another particular embodiment, the genotoxic agent is UV light. The skilled person will appreciate that the UV light that is used for the treatment of the polynucleotide must be sufficient so as to substantially damage the capacity of the polynucleotide to produce a functional fluorescent protein but not to inactivate the polynucleotide beyond the DNA repair capacity of a cell. The amount of light can then be determined by standard experimentation. An adequate range of irradiation has been experimentally proven to be 700-1200 J/m$^2$.

Knowing the DRC of a cell in an accurate way, the physician can predict if the response of a subject suffering from cancer to a chemotherapy treatment directed to DNA damage is going to be positive for the subject.

Thus, in another aspect, the invention relates to an *in vitro* method for determining the genetic susceptibility to cancer of a subject comprising

(i) determining the DNA repair capacity of a cell from said subject according to the first method of the invention or of a cell population from said subject by a method according to the second method of the invention, and
(ii) comparing the DNA repair capacity values obtained in step (i) with standard reference values

wherein a DNA repair capacity lower than the DNA repair capacity of the reference values is indicative of high susceptibility to cancer.

In another aspect, the invention relates to an *in vitro* method for predicting the response of a subject suffering from cancer to a chemotherapy treatment directed to damaging DNA comprising

(i) determining the DNA repair capacity of a cell from said subject according to the first method of the invention or of a cell population from said subject by a method according to the second method of the invention, and
(ii) comparing the DNA repair capacity obtained in step (i) with standard reference values

wherein a DNA repair capacity values lower than the DNA repair capacity of the reference values is indicative of a better response of the subject to the chemotherapy directed to damaging DNA.

As used herein, "standard reference values" is understood as the median value of DRC measured in a collection of cells from cancer patients.

Chemotherapeutic agents directed to damaging the DNA are those agents capable of provoking at least one lesion in the DNA molecule. Typical lesions include base loss, base modification (deamination, oxidation, alkylation), formation of thymidine dimmers, double-strand breaks, DNA protein cross-links, inter-strand cross-links and the like. By way of example, agents capable of inducing DNA damage include, without limitation, (i) alkylating agents, such as Cyclophosphamide (cycloblastin, endoxan), Ifosphamide, Melphalan, Chlorambucil (leukeran), BCNU, CCNU, Decarbazine, Procarbazine, Busulfan, Thiotepa, carmustine, lomustine, platinum-based compounds (cisplatin, carboplatin, Oxaliplatin), etoposide (VP-16), etc.; (ii) Anthracyclines, such as Daunorubicin, Doxorubicin, Idarubicin, Epirubicin, Mitoxantrone, etc.; (iii) purine antagonists such as 6-mercaptopurine (Puri-Nethol), 6-thioguanine, dacarbazine, fludurabine, etc.; and (iv) Pyrimidine antagonists such as 5-fluorouracil (Efudix), cytarabine, capecitabine (Xeloda), gemcitabine (Gemzar) etc.

In another aspect, the invention relates to a method for the screening of compounds useful for stimulating/inhibiting the DNA repair capacity comprising:

(i) introducing into a cell a polynucleotide encoding in its natural state a fluorescent protein, wherein said polynucleotide has been previously contacted with a genotoxic agent so that the protein can not perform its function,
(ii) contacting the cell obtained in step (i) with a candidate compound,
(iii) maintaining the cell under conditions adequate for expression of the polynucleotide and
(iv) determining the intensity of the fluorescence emitted by the cell due to said fluorescent protein and comparing it with the fluorescence emitted by a control cell which has not been contacted with the candidate compound

wherein

(i) if the intensity of the fluorescence emitted by the cell is higher than the fluorescence emitted by the control cell, then the candidate compound is able to stimulate the DNA repair capacity of a cell, or
(ii) if the intensity of the fluorescence emitted by the cell is lower than the fluorescence emitted by the control cell, then the candidate compound is able to inhibit the DNA repair capacity of a cell.

In another aspect, the invention relates to a method for the screening of compounds useful for stimulating/inhibiting the DNA repair capacity comprising:

(i) introducing into a cell population a polynucleotide encoding in its natural state a fluorescent protein, wherein said polynucleotide wherein said polynucleotide has been previously contacted with a genotoxic agent so that the polynucleotide has been rendered unable of expressing a functional fluorescent protein,
(ii) contacting the cell population obtained in step (i) with a candidate compound,
(iii) maintaining the cell population under conditions adequate for expression of the polynucleotide and
(iv) determining the percentage of cells which produce fluorescence due to said fluorescent protein above a threshold fluorescence intensity level and comparing it with the percentage of cells which produce fluorescence due to said fluorescence protein of a cell population which has not been contacted with the candidate compound

wherein

(i) if the percentage of cells emitting fluorescence over a threshold value is higher than the percentage of cells emitting fluorescence over a threshold value in the control cell population, then the candidate compound is able to stimulate the DNA repair capacity of a cell, or
(ii) if the percentage of cells emitting fluorescence over a threshold value is lower than the percentage of cells emitting fluorescence over a threshold value in the control cell population, then the candidate compound is able to inhibit the DNA repair capacity of a cell.

In the screening methods according to the present invention, steps (i), (iii) and (iv) are carried out as essentially described for the first and second method of the invention.

Step (ii) comprises the step of contacting a cell or a cell population with a compound whose capacity to stimulate or inhibit the DNA repair capacity of said cell or cell population wants to be assessed. According to the present invention, "putting in contact" a cell or a cell population with the candidate compound includes any possible way of taking the candidate compound inside the cell or cell population. Thus, in the event that the candidate compound is a molecule with low molecular weight, it is enough to add said molecule to the culture medium. In the event that the candidate compound is a molecule with a high molecular weight (for example, biological polymers such as a nucleic acid or a protein), it is necessary to provide the means so that this molecule can access the cell interior. In the event that the candidate molecule is a nucleic acid, conventional transfection means can be used, as described previously for the introduction of the DNA construct. In the event that the candidate compound is a protein, the cell can be put in contact with the protein directly or with the nucleic acid encoding it coupled to elements allowing its transcription / translation once they are in the cell interior. To that end, any of the aforementioned methods can be used to allow its entrance in the cell interior. Alternatively, it is possible to put the cell in contact with a variant of the protein to be studied which has been modified with a peptide which can promote the translocation of the protein to the cell interior, such as the Tat peptide derived from the HIV-1 TAT protein, the third helix of the Antennapedia homeodomain protein from *D.melanogaster*, the VP22 protein of the herpes simplex virus and arginine oligomers (Lindgren, A. et al., 2000, Trends Pharmacol. Sci, 21:99-103, Schwarze, S.R. et al., 2000, Trends Pharmacol. Sci., 21:45-48, Lundberg, M et al., 2003, Mol. Therapy 8:143-150 and Snyder, E.L. and Dowdy, S.F., 2004, Pharm. Res. 21:389-393).

The compound to be assayed is preferably not isolated but forms part of a more or less complex mixture derived from a natural source or forming part of a library of compounds. Examples of libraries of compounds which can be assayed according to the method of the present invention include, but are not limited to, libraries of peptides including both peptides and peptide analogs comprising D-amino acids or peptides comprising non-peptide bonds, libraries of nucleic acids including nucleic acids with phosphothioate type non-phosphodiester bonds or peptide nucleic acids, libraries of antibodies, of carbohydrates, of compounds with a low molecular weight, preferably organic molecules, of peptide mimetics and the like. In the event that a library of organic compounds with a low molecular weight is used, the library can have been preselected so that it contains compounds which can access the cell interior more easily. The compounds can thus be selected based on certain parameters such as size, lipophilicity, hydrophilicity, capacity to form hydrogen bonds.

The compounds to be assayed can alternatively form part of an extract obtained from a natural source. The natural source can be an animal, plant source obtained from any environment, including but not limited to extracts of land, air, marine organisms and the like.

The following example illustrates the invention and should not be considered in a limiting sense, but rather in an illustrative sense of the invention.

## EXAMPLES

**Determining the DNA repair capacity of a cell**

I. <u>MATERIAL AND METHODS</u>

### *Host cell reactivation assay (HCRA)*

15-20 ml of whole blood collected in sodium heparin (depending on the hemogram) was mixed in a 50 ml Falcon tube. Subsequently, one volume (15-20 ml) of RPMI 1640 culture media was added to whole blood and mixed by inversion for six to eight times. The resulting mixture of whole blood and culture media was extended carefully over one volume (15-20 ml) of Ficoll-Paque Plus layer (Amersham Pharmacia cat #: 17-1440-03), without disturbing the interphase, and centrifuged at 600g for 25 minutes at 22 °C with low brake. After removing the samples from the centrifuge, the peripheral blood leukocytes (PBL) interphase was carefully removed, transferred into a clean Falcon 50 ml tube (completed culture media was added up to 35 ml), gently mixed. 90 μl of cell mixture were then transferred into 1.5 ml tubes.

Subsequently, 90 μl of Tripan blue (0,4%) was mixed with 90 μl of cell suspension and counted using a Neubauer chamber. The cells were centrifuged at 1.000 rpm for 15 minutes at room temperature and the supernatant was discarded by inversion. The cells were resuspended in ice-cold freezing media (1 ml of Freezing media each for each $6 \times 10^6$ cells), said media consisting of 90% FBS and 10% DMSO. The cells were cryopreserved at -80 °C by introducing the cryotubes containing said cells into ice-cold (4°C) 2-propanol to generate a decrease temperature ramp of 1°C/minute in order to avoid temperature shock. Once the cells reached the -80°C temperature, the samples were preserved at -80°C or transferred into liquid nitrogen.

### *Thawing cells for HCRA experiment*

Experimental Day 1

To thaw the cells, the tubes containing the cells were directly transferred from -80°C into ice, and then mixed by pippeting the cells from the cryotube into a conic 15 ml tube containing 9 ml of complete DMEM- CM. The conic tube was vigorously mixed by inversion in order to obtain a homogeneous cell suspension and then. An aliquot (90 $\mu$l) from said suspension was mixed with 90 $\mu$l Trypan blue (0,4%) and then, the number of cells was counted under Neubauer chamber.

The cells were then splitted in complete RPMI 1640 comlete culture media with 56 $\mu$g of PHA/ml at a concentration of 0,4-0,5x10$^6$ cells/ml and cultured for 48-96 hours at 37°C in 5% CO2 atmosphere.

Experimental Day 5.

NOTE II: An aliquot of complete DMEM + 4.5 g/L of glucose culture medium (15% FBS, 1x Antibiotics and L-glutamine) (DMEM-CM), was prepared at 37°C prior to starting the experiment. It was also important to prepare a 24 well plate with 1.8 ml of DMEM + 4,5 g/L of glucose complete culture media, in each well. The plate must be pre-warmed at 37°C without PHA stimulation in the media.

All the reagents for transfection were pre-warmed at room temperature (plasmids and nucleofection solution).

In each experimental procedure, one Xeroderma pigmentosum lymphoblastic cell line (GM2345 as XPA$^{-/-}$ or GM2246 as XPC$^{-/-}$) was used as negative control. In addition, one apparently wild type lymphoblastic cell line (GM7544 or GM3798) was used as positive control of DNA repair capacity.

After incubation, the number of cells was counted again as explained above.

At least 4x10$^6$ Cells were used for determining the DNA repair capacity. A sample of the cell suspension corresponding to 5-6x10$^6$ cells was transferred into a new conic clean tube. The cells were then centrifuged at 1.200 rpm for 15 minutes at 22°C. The supernatant was carefully removed by inversion. The remaining traces of culture media were eliminated by aspirating with a pipette connected to a vacuum pump, without disturbing the cell pellet. The cell pellet was resuspended in nucleofection solution (Human T Cell 96-well Nucleofector Kit; Amaxa; cat number: VHPA-1002) at 10$^6$ cells/20 $\mu$l, taking care of avoiding long time exposure of cells to nucleofection solution.

A sample comprising 2 x10$^6$ cells (40 $\mu$l of cell suspension) was mixed with 400 ng of wild type pmax C+ plasmid (Human T Cell 96-well Nucleofector Kit; Amaxa; cat number: VHPA-1002). The remaining 2x10$^6$ cells from the same sample were mixed with damaged-DNA pmax C+ plasmid (for instance UV 800J/m$^2$ as described in the UV DNA damage protocol).

Each aliquot was mixed gently by pipetting. From each sample (undamaged plasmid vs. damaged plasmid), 22 $\mu$l were then transferred into two different wells (replicates) of the nucleofector 96 well plate. Immediately, the transfection of the cells was carried out by using the following Nucleofection conditions:

- Cell lines: EH-100
- Peripheral Blood Lymphocytes (PBLs): FF-113

A non-transfection sample (10$^6$ cells) either from the cell lines or from peripheral blood leukocytes were used to establish the threshold of EGFP+ detection in the Flow Cytometer.

Immediately after transfection, 180 $\mu$l of DMEM-CM culture medium pre-warmed at 37°C (see NOTE II above) was added to each well. Subsequently, each cell suspension was transferred into a well of a 24-well plate (containing 1.8 ml of 37°C pre-warmed culture media) to reach a final volume of 2 ml. 24 hours after transfection, the ratio of the EGFP+ cells with respect to EGFP- cells was determined by flow cytometry both in undamaged and in damaged plasmid samples.

As reference, a result eligible for DRC, should derive from a sample which could not have higher coefficient of variation of 15% in replicates of the same experimental conditions (see two replicates for undamaged or damaged plasmid).

### *Plasmid DNA-UV Damage protocol*

The plasmid DNA concentration was measured with spectrophotometer and adjusted to a concentration at 200 ng/$\mu$l. Aliquots of 500 $\mu$l (1 $\mu$g) were prepared in a 24 well plate. The plate was then irradiated at 700 to 1200 J/m2.

II- <u>RESULTS</u>

As shown in figure 1, FACS analysis of non-transfected sample peripheral blood leukocytes (PBLs) using forward and side scatter allows the identification of three different cell populations including: non-viable cells showing high side-scatter and viable cells, including monocytes and lymphocytes.

The sorted viable cells were then analysed by two-dimensional FACS using FL2H and EGFP filters. The non-transfected sample was used to draw a threshold value which allows to separate EGFP+ cells from EGFP- cells (Figure 2).

From one patient, $2x10^6$ PBL cells were transfected with the wild type plasmid. FACS sorting as described in the second paragraph was used to separate alive and death cells. Only alive cells were then analysed by FACS scan was used in order to discriminate the percentage of EGFP+ cells from the EGFP- population, by using EGFP and FL2H filters. The resulting FACS scan shows (Figure 3) that 27% of the PBLs population was negative for EGFP (i.e. it does not display fluorescence and appears in the scan below the threshold value) whereas 73% of the population was positive to EGFP (i.e. it displays fluorescence and is found above the threshold value).

The remaining $2x10^6$ PBL cells were transfected with the UV-light damaged-DNA plasmid at 800 J/m2 containing the coding sequence of the EGFP gene. The viable cells selected by FACS sorting as described in the second paragraph were used to separate alive and death cells. Alive cells were then analysed by FACS scan in order to discriminate the percentage of EGFP+ cells from the EGFP- population, by using EGFP and FL2H filters. FACS scan shows (Figure 4) that 70% of the PBLs population was negative to EGFP (i.e. it does not display fluorescence) whereas the 30% of the population was positive to EGFP (i.e. it displays fluorescence). With these data, the DNA repair capacity of the cell was calculated as the ratio of positive EGFP population value to the negative EGFP population value, multiplied by 100%.

$$\frac{30\%\ (800\,J/m2)}{73\%\ (0\,J/m2)} \times 100\% = 41,09\%\ DRC$$

**Claims**

1. An *in vitro* method for determining the DNA repair capacity of a cell comprising:

   (i) introducing into said cell a polynucleotide encoding in its natural state a fluorescent protein, wherein said polynucleotide has been previously contacted with a genotoxic agent so that the polynucleotide has been rendered unable of expressing a functional fluorescent protein;
   (ii) maintaining the cells under conditions adequate for expression of said polynucleotide and
   (iii) determining the fluorescent emission of the cell due to the fluorescent polypeptide

   wherein the intensity of the fluorescence emission is indicative of the DNA repair capacity of the cell.

2. Method according to claim 1, wherein the polynucleotide is introduced into the cell by electroporation, preferably by nucleofection.

3. Method according to claims 1 or 2, wherein the fluorescence detection is carried out by flow cytometry.

4. Method according to anyone of claims 1 to 3, wherein the fluorescent protein is EGFP (Enhanced Green Fluorescent Protein).

5. An *in vitro* method for determining the DNA repair capacity of a cell population comprising:

   (i) introducing into said cell population a polynucleotide encoding in its natural state a fluorescent protein, wherein said polynucleotide has been previously contacted with a genotoxic agent so that the polynucleotide has been rendered unable of expressing a functional fluorescent protein;
   (ii) maintaining the cells under adequate conditions for expression of said polynucleotide and
   (iii) determining the percentage of cells emitting fluorescence due to the fluorescent polypeptide above a threshold fluorescence value,

wherein the threshold fluorescence value is the value of a control cell population which does not contain a polynucleotide coding for a fluorescent protein and

wherein the percentage of cells emitting fluorescence over said threshold level is indicative of the DNA repair capacity of the cell population.

6. Method according to claim 5 wherein step (iii) comprises determining the ratio of the percentage of cells emitting fluorescence above a given threshold value and the percentage of cells emitting fluorescence below a given threshold value and wherein said ratio is indicative of the DNA repair capacity of the cell population.

7. Method according to claims 5 or 6, wherein the cell population used in step (i) has been selected to contain only viable cells.

8. Method according to any of claims 5 to 7, wherein the polynucleotide is introduced into the cell by electroporation, preferably by nucleofection.

9. Method according to any of claims 5 to 8, wherein the fluorescence detection is carried out by flow cytometry.

10. Method according to any of claims 5 to 9, wherein the fluorescent protein is EGFP (Enhanced Green Fluorescent Protein).

11. An isolated polynucleotide encoding in its natural state a fluorescent protein,
wherein said polynucleotide has been contacted with a genotoxic agent so that (i) the polynucleotide has been rendered unable to express a functional fluorescent protein and (ii) the polynucleotide comprises, at least, one thymidine dimer.

12. Polynucleotide according to claim 11, wherein the fluorescent protein is EGFP.

13. Polynucleotide according to claims 11 or 12, wherein the genotoxic agent is UV light.

14. An *in vitro* method for determining the genetic susceptibility to cancer of a subject comprising

   (i) determining the DNA repair capacity of a cell from said subject according to anyone of claims 1 to 4 or of a cell population from said subject by a method according to anyone of claims 5 to 10, and
   (ii) comparing the DNA repair capacity values obtained in step (i) with standard reference values

   wherein a DNA repair capacity lower than the DNA repair capacity of the reference values is indicative of high susceptibility to cancer.

15. An *in vitro* method for predicting the response of a subject suffering from cancer to a chemotherapy treatment directed to damaging DNA comprising

   (i) determining the DNA repair capacity of a cell from said subject according to anyone of claims 1 to 4 or of a cell population from said subject by a method according to anyone of claims 5 to 10, and
   (ii) comparing the DNA repair capacity obtained in step (i) with standard reference values

   wherein a DNA repair capacity values lower than the DNA repair capacity of the reference values is indicative of a better response of the subject to the chemotherapy directed to damaging DNA.

FIGURE 1

# Non-transfected PBLs

This sample it is used to stablish the threshlod for EGFP+ cells, only in alive cells.

All  EGFP- PBL cells

0% of EGFP+ PBL cells

EGFP+

FIGURE 2

# Undamaged plasmid transfected PBLs

Undamaged
plasmid.

27% of EGFP- PBL
cells

73% of EGFP+ PBL
cells

FIGURE 3

# UV light damaged plasmid transfected PBLs

UV damaged 800 J/m2 plasmid.

70% of EGFP- PBL cells

30% of EGFP+ PBL cells

$$\frac{30\%\ (800\,J/m2)}{73\%\ (0\,J/m2)} \times 100\% = 41,09\%\ DRC$$

FIGURE 4

European Patent Office

**EUROPEAN SEARCH REPORT**

Application Number

EP 08 38 0156

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ATANASSOV BOYKO S ET AL: "Relationship between DNA repair capacity and resistance to genotoxins in four human cell lines." CANCER DETECTION AND PREVENTION 2003, vol. 27, no. 1, 2003, pages 24-29, XP002492741 ISSN: 0361-090X | 1,4,5,7, 10-13,15 | INV. C12Q1/02 G01N33/50 |
| Y | | 2,3,6,8, 9,14 | |
| | * abstract * * page 25, column 1, line 26 - page 25, column 1, line 33 * * page 25, column 2, paragraph 2.2 - page 26, column 1, paragraph 2.3 * * page 26, column 2, line 13 - page 26, column 2, line 19 * * page 26, column 2, line 45 - page 26, column 2, line 50 * ----- | | |
| X | ROGUEV A ET AL: "Two-wavelength fluorescence assay for DNA repair." ANALYTICAL BIOCHEMISTRY 15 DEC 2000, vol. 287, no. 2, 15 December 2000 (2000-12-15), pages 313-318, XP002492742 ISSN: 0003-2697 | 1,4,5, 10-13 | |
| Y | | 2,3,6,8, 9,14,15 | TECHNICAL FIELDS SEARCHED (IPC) G01N C12Q |
| | * abstract * * page 314, column 1, line 18 - page 314, column 1, line 28 * * page 314, column 1, line 52 - page 314, column 2, line 27 * * page 314, column 2, line 45 - page 315, column 1, line 30 * ----- -/-- | | |

The present search report has been drawn up for all claims

| Place of search | Date of completion of the search | Examiner |
|---|---|---|
| Munich | 1 September 2008 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 08 38 0156

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BERWICK M ET AL: "Markers of DNA repair and susceptibility to cancer in humans: an epidemiologic review." JOURNAL OF THE NATIONAL CANCER INSTITUTE 7 JUN 2000, vol. 92, no. 11, 7 June 2000 (2000-06-07), pages 874-897, XP002492743 ISSN: 0027-8874 * page 874, column 1, line 39 - page 874, column 2, line 5 * * page 893, column 1, line 12 - page 893, column 1, line 21 * | 1,14 | |
| Y | SLEBOS R J C ET AL: "A novel host cell reactivation assay to assess homologous recombination capacity in human cancer cell lines" BIOCHEMICAL AND BIOPHYSICAL RESEARCH COMMUNICATIONS, ACADEMIC PRESS INC. ORLANDO, FL, US, vol. 281, no. 1, 16 February 2001 (2001-02-16), pages 212-219, XP002227693 ISSN: 0006-291X * abstract * * page 213, column 2, line 3 - page 213, column 2, line 7 * * page 214, column 1, line 57 - page 214, column 1, line 62; figure 2 * | 3,4,6,9 | TECHNICAL FIELDS SEARCHED (IPC) |

-/--

The present search report has been drawn up for all claims

| Place of search | Date of completion of the search | Examiner |
|---|---|---|
| Munich | 1 September 2008 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

**EUROPEAN SEARCH REPORT**

Application Number

EP 08 38 0156

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LEE ET AL: "Monitoring repair of DNA damage in cell lines and human peripheral blood mononuclear cells" ANALYTICAL BIOCHEMISTRY, ACADEMIC PRESS INC. NEW YORK, vol. 365, no. 2, 10 May 2007 (2007-05-10), pages 246-259, XP022077174 ISSN: 0003-2697 * page 249, column 2, line 9 - page 249, column 2, line 36 * * page 254, column 2, line 9 - page 254, column 2, line 12 * * page 256, column 1, line 21 - page 256, column 2, line 2 * | 2-4,6,8, 9 | |
| A | ATHAS W F ET AL: "Development and field-test validation of an assay for DNA repair in circulating human lymphocytes." CANCER RESEARCH 1 NOV 1991, vol. 51, no. 21, 1 November 1991 (1991-11-01), pages 5786-5793, XP002492744 ISSN: 0008-5472 | 1-15 | |
| Y | * abstract * * page 5786, column 2, line 46 - page 5786, column 2, line 49 * * page 5787, column 2, line 5 - page 5787, column 2, line 14 * * page 5791, column 2, line 23 - page 5791, column 2, line 25; tables 6,7 * * page 5789, column 2, line 23 - page 5789, column 2, line 36 * | 15 | TECHNICAL FIELDS SEARCHED (IPC) |

The present search report has been drawn up for all claims

| Place of search | Date of completion of the search | Examiner |
|---|---|---|
| Munich | 1 September 2008 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4975365 A

### Non-patent literature cited in the description

- **Qiao, Y. et al.** *Mutation Research,* 2002, vol. 509, 165-175
- **Laget, M et al.** *Path Biol.,* 1995, vol. 43, 596-600
- **Ausubel, F.M. et al.** Current Protocols in Molecular Biology. John Wiley & Sons Inc, 2003
- **M.L. Roach ; J.D. McNeish.** *Methods in Mol. Biol.,* 2002, vol. 185, 1
- **Hess et al.** *Nucleic Acid Res.,* 1996, vol. 24, 824-828
- **Pasheva et al.** *Int. J. Biochem. Cell Biol.,* 2002, vol. 34, 87-92
- **Laws et al.** *Mut. Res.,* 2001, vol. 484, 3-18
- **Zhang et al.** *Mol. Cell. Biol.,* 2002, vol. 22, 2388-2397
- **Dasaradhi et al.** *Chem. Res. Tox.,* 1997, vol. 10, 189-196
- **Steinkamp J. et al.** *Journal of Immunological Methods,* 1999, vol. 226 (1-2), 59-70
- **Derby E et al.** *Immunol Lett.,* 01 August 2001, vol. 78 (1), 35-9
- **Douki, T. et al.** *J. Biol. Chem.,* 2000, vol. 275, 1167-11685
- **Ramaiah, D. et al.** *Nucleic Acids Research,* 1998, vol. 26, 3940-3943
- **Komatsu, Y. et al.** *Nucleic Acids Research,* 1997, vol. 25, 3889-3894
- **Lund, V. et al.** *Nucleic Acids Research,* 1989, vol. 17, 539-551
- **Lindgren, A. et al.** *Trends Pharmacol. Sci,* 2000, vol. 21, 99-103
- **Schwarze, S.R. et al.** *Trends Pharmacol. Sci.,* 2000, vol. 21, 45-48
- **Lundberg, M et al.** *Mol. Therapy,* 2003, vol. 8, 143-150
- **Snyder, E.L. ; Dowdy, S.F.** *Pharm. Res.,* 2004, vol. 21, 389-393